(19) Europäisches Patentamt

European Patent Office

Office européen des brevets

(11) **EP 1 300 140 B1**

(12) **EUROPEAN PATENT SPECIFICATION**

(45) Date of publication and mention
of the grant of the patent:
**30.11.2005 Bulletin 2005/48**

(51) Int Cl.⁷: **A61K 9/22**, A61K 31/498,
A61P 25/24

(21) Application number: **01670006.4**

(22) Date of filing: **08.10.2001**

(54) **Long release matricial formulations of pirlindol hydrochloride**

Pirlindolhydrochlorid-Matrixformulierungen mit verlängerter Wirkstofffreigabe

Formulations matricielles à libération prolongée contenant du pirlindole hydochloride

(84) Designated Contracting States:
**AT BE CH CY DE DK ES FI FR GB GR IE IT LI LU
MC NL PT SE TR**

(43) Date of publication of application:
**09.04.2003 Bulletin 2003/15**

(73) Proprietor: **TECNIMEDE-SOCIEDADE
TECNICO-MEDICINAL, S.A.**
**2685-338 Prior Velho (PT)**

(72) Inventors:
 • **Mendes Cerdeira, Ana Maria**
 **2700-098 Amadora (PT)**
 • **De Sousa Goucha Jorge, Pedro Manuel**
 **2670 Loures (PT)**

(74) Representative: **Ferreira, Maria Silvina et al**
**CLARKE MODET & Co.,**
**Rua Castilho, 50-5**
**1269/163 Lisboa (PT)**

(56) References cited:
**GB-A- 1 340 528**

 • **BRUHWYLER J ET AL: "Pirlindole: A selective
 reversible inhibitor of monoamine oxidase A. A
 review of its preclinical properties."
 PHARMACOLOGICAL RESEARCH, vol. 36, no. 1,
 July 1997 (1997-07), pages 23-33, XP002193389
 ISSN: 1043-6618**

## Description

### Field of the invention

The invention refers to the obtention of long release matricial pharmaceutical forms comprising pirlindol hydrochloride that allow the attainment of similar dissolution profiles in mediums with different pH values.

### Description of prior art

Pirlindol hydrochloride, also named pyrazidol, is an antidepressant drug that has the molecular formula $C_{15}H_{18}N_2HCl$ and a molecular weight of 345.42.

The half-life time ($t_{1/2}$) of pirlindol hydrochloride ranges from 5 to 6 hours and the time after which a maximum concentration is achieved (t max) from 1 to 2 hours. The advised therapeutic doses range from 100 and 300 mg/day.

Antidepressant therapies often require long-term periods. In patients with psychiatric problems a single take has advantages, since this group of patients forgets to take its medication regularly (Rubinstein, M.H., In: Aulton, M. E., 1988, pages 304-322).

Therefore, the characteristics of pirlindol hydrochloride and the therapy for which it is destined, make this active substance a good candidate for oral administration, via long release pharmaceutical forms.

When a conventional pharmaceutical form is orally administrated (instant release), the plasmatic concentration of the drug rapidly rises, until a maximum level is attained, then decreasing slowly while the drug is metabolised or excreted. A long release pharmaceutical form, as claimed for pirlindol hydrochloride, allows the maintenance of the plasmatic concentration of the drug in the range of a minimum effective concentration and a maximum toxic concentration, fact that improves the therapeutic effectiveness of the drug and reduces the toxic effects.

Using a long release system, more or less constant release levels of the drug may be achieved; therefore a smaller amount of the drug will be necessary (relatively to conventional pharmaceutical forms) to produce a particular duration of the effect. Long release pharmaceutical forms are particularly useful when the half-life time of the drug is less than the period of duration of the effect aspired.

This may result in an increased adherence of the patient to the therapy, relatively to the multiple doses used for the conventional forms (Baker, R. In: John Wiley & Sons, 1987. Controlled Release Of Biologically Active Agents, pages 4-21).

There are several types of controlled release systems:

- Coated systems;
- Matricial systems;
- Biodegradable systems;
- Osmotic systems;
- Mechanic pumps.

In a matricial system, subject of this invention, the drug is homogeneous disperse in a polymeric matrix. So, in the case of a tablet, this may be divisible, which does not occur in the case of coated systems. The fact of the tablet being divisible allows adjusting the dose according to the needs of the patient.

Beside the advantages inherent to these systems, long release tablets have in addition the technological advantages of the conventional pharmaceutical forms, since they do not require specific technology.

In the long release matricial or coated pharmaceutical forms of the type of tablets, it is very important that the dissolution profiles are equivalent in mediums of different pH values. This is due to the gastrointestinal residential times, considering a normal population, which may range from 5 to 36 hours (mean time of 24 hours), being in that period of time the tablet located in mediums of different pH values. In case of the dissolution profiles being not similar in mediums of different pH values, it may exist a great variability intra and inter-individual (Barr, W.H., and Skelly, J.P. In: Robinson, J.R. and Lee, V.H.L., 1987, Controlled drug delivery, pages 253-291).

There are numerous polymers that allow prolonging the release. Examples are cellulose derivatives, such as sodium carboxymethyl cellulose or hydroxipropylmethyl cellulose, methacrylic acid derivatives, such as Eudragit NE 30D® or Eudragit RS PO®. This polymers, when applied in long release pharmaceutical forms of the matricial type and active substances with the characteristics of pirlindol hydrochloride, yield appropriate dissolution profiles in acid medium (pH 1,2), but with a very slow dissolution rate in alkaline or near neutral medium (for example pH 6,8). This essentially results in a reduced solubility of pirlindol hydrochloride in mediums with more elevated pH, preventing therefore the release to occur in a similar fashion at different pH values.

The European patent 0484106 refers the necessity of the association of sodium or magnesium salts of antiacids to soluble sodium alginate derivatives in order to reduce de dissolution rate of an active substance.

The International patent application WO 98/56357. describes a long release formulation for alkaline substances with very small solubility. This patent states the necessity of associating to a hydrosoluble alginate salt an alginic acid complex salt and an organic carboxylic acid, to allow an increase of solubility of the active substance during the course through the gastrointestinal system and so keep similar the release at different pH values.

The International patent application WO 99/29305 refers the need to associate three polymers with distinct characteristics in order to obtain similar dissolutions in mediums with different pH values.

GB-1340528-A discloses a tablet comprising pirlindol hydrochloride.

However, when the pirlindol hydrochloride is associated to sodium alginate, in order to obtain long release matricial tablets by the wet granulation process, an increase of the rate of release of this active substance during the storage period is verified.

## Summary of the invention

The object of the present invention is the obtention of long release matricial pharmaceutical forms of pirindol hydrochloride that have stable and similar dissolution profiles in mediums of different pH values (1.2<pH<8.0).

The present invention consists in the obtention of pharmaceutical formulations by the dry mixing and/or granulation process of the pirlindol hydrochloride with alginic acid derivative polymers (for example sodium alginate, calcium alginate) and commonly used excipients in the pharmaceutical technology.

Further, the present invention consists in the obtention of pharmaceutical formulations composed by a pirlindol hydrochloride and sodium alginate matrix, stable during the storage period and that show good dissolution, absorption properties and consequently a adequate bioavailability when orally administrated.

## Brief description of the drawings

FIGURE 1: Dissolution profiles in acid medium and alkaline medium for a long release tablet formulation of pirlindol hydrochloride comprising sodium alginate (7.33%, w/w).

FIGURE 2: Comparison of the dissolution profiles (HCl 0.1N, pH 1.2) at time 0 and on the 26th day.

FIGURE 3: Topographic characterization of the dissolution as a function of time and pH.

## Detailed description of the invention

The aim of the present invention is the obtention of long release tablets of pirlindol hydrochloride that have stable and similar dissolution profiles at all pH values of the gastrointestinal system. Therefore, the pirlindol hydrochloride is associated to an alginic acid derivative by dry mixing and/or granulation process followed by compression.

It is also a characteristic of the present invention, the necessity of using only one polymer to control the release of pirlindol hydrochloride, not being necessary the inclusion of other additives (for example organic acids).

The alginic acid derivatives, such as sodium or calcium alginate, are converted into alginic acid in acid medium and in alkaline medium into sodium alginate. This fact in association with the characteristics of pirlindol hydrochloride allows the obtention of similar dissolution profiles in mediums of different pH values.

The alginic acid derivative may show several viscosity degrees and it should be selected according to the whished dissolution profile.

According to a preparation manner, the pirlindol hydrochloride is mixed with technologically appropriate excipients for compression (for example lactose, microcrystalline cellulose). After an adequate mixing period, the sodium alginate is added and it is mixed again for a new period of time. After this phase, lubricants are added if necessary and it is compressed. The tablets may be use as such or divided and compressed again or divided, calibrated and used for the filling of capsules.

When appropriate excipients are added, the mixtures of pirlindol hydrochloride with the alginic acid derivatives may further be used for the formulation of pellets.

In the following examples of obtention processes of long release matricial pharmaceutical forms of pirlindol hydrochloride with alginic acid derivatives are shown.

**Comparative Example 1:** Preparation of long release tablets, dosed for 100 mg of pirlindol hydrochloride with sodium alginate (6.20%, w/w).

## 1 - COMPOSITION

The unitary qualitative and quantitative composition for pirlindol hydrochloride tablets dosed for 100 mg, obtained by the wet granulation process, is shown in Table 1.

TABLE 1 -

| Unitary qualitative and quantitative composition for pirlindol hydrochloride tablets dosed for 100 mg, obtained by the wet granulation process | | |
|---|---|---|
| **Composition** | **Amount (mg)** | **Function** |
| Pirlindol.HCl | 100.00 | Active substance |
| Sodium alginate | 14.90 | Polymer for prolonging the release |
| Microcrystalline cellulose | 122.80 | Diluent |
| Magnesium stearate | 2.80 | Lubricant |
| **Mass of 1 tablet** | **240.5** | -- |

## 2- PREPARATION METHOD

Briefly, the pirlindol hydrochloride is mixed with the microcrystalline cellulose and part of the sodium alginate. This powder mixture is granulated with a 3% (w/w) sodium alginate solution. The obtained granulate is dried in a stationary bed oven at 40°C. After the addition of the external phase (magnesium stearate), the compression is accomplished by using circular concave punches, without slit (9 mm - 100 mg tablets).
The tablets obtained were packed in blisters of OPA/ALU complex. The final product was stored at different humidity and temperature conditions:

- Exsicator 85% RH
- Oven at 25°C/60% RH
- Oven at 40°C/75% RH
- Oven at 60°C

## 3- DISSOLUTION PROFILES

The dissolution profiles of the tablets submitted to different storage conditions were carried out in HCl 0.1N. The results are shown in Table 2.

TABLE 2 -

| Results of the dissolution studies (HCl 0.1 N, pH 1.2) of the tablets submitted to different stability conditions | | | | | |
|---|---|---|---|---|---|
| Time (h) | Released % | | | | |
| | 25°C/60% RH*, $t_{0d}$** | 25°C/60% RH, $t_{52d}$*** | 85% RH, $t_{52d}$ | 40°C/75% RH, $t_{52d}$ | 60°C, $t_{52d}$ |
| **0.0** | 0.0 | 0.0 | 0.0 | 0.0 | 0.0 |
| **0.3** | 11.8 | 14.2 | 16.5 | 20.6 | 28.5 |
| **0.5** | 17.8 | 22.4 | 25.6 | 32.8 | 49.8 |
| **0.8** | 21.9 | 27.8 | 32.0 | 40.9 | 63.0 |
| **1.0** | 25.7 | 32.2 | 37.5 | 47.3 | 71.1 |
| **1.5** | 32.5 | 40.0 | 46.3 | 56.8 | 80.7 |

*RH = Relative humidity;
**$t_{0d}$ = Analyses at time zero;
***$t_{52d}$ = Analyses on the 52nd day.

TABLE 2 - (continued)

| Time (h) | Released % | | | | |
|---|---|---|---|---|---|
| | 25°C/60% RH*, $t_{0d}$** | 25°C/60% RH, $t_{52d}$*** | 85% RH, $t_{52d}$ | 40°C/75% RH, $t_{52d}$ | 60°C, $t_{52d}$ |
| 2.0 | 38.1 | 47.1 | 53.7 | 65.4 | 86.6 |
| 3.0 | 47.2 | 59.3 | 66.3 | 78.3 | 93.5 |
| 4.0 | 54.9 | 69.6 | 75.4 | 88.1 | 97.4 |
| 5.0 | 61.3 | 77.5 | 82.2 | 93.8 | 99.1 |
| 6.0 | 66.7 | 86.7 | 87.0 | 97.3 | 99.8 |
| 7.0 | 71.5 | 87.9 | 90.8 | 99.1 | 100.1 |
| 8.0 | 75.7 | 91.6 | 93.6 | 100.1 | 100.2 |
| 9.0 | 79.3 | 95.1 | 95.8 | 100.7 | 100.3 |
| 10.0 | 82.5 | 97.2 | 97.5 | 101.1 | 100.5 |
| 11.0 | 86.0 | 98.5 | 98.7 | 101.3 | 100.7 |
| 12.0 | 89.3 | 99.5 | 99.5 | 101.5 | 100.8 |

*RH = Relative humidity;

**$t_{0d}$ = Analyses at time zero;

***$t_{52d}$ = Analyses on the 52nd day.

The release rate of the pirlindol hydrochloride from the matricial tablets obtained by the wet granulation process increases with the storage time, independently of the storage conditions.

**Example 2:** Preparation of long release tablets, dosed for 100 mg of pirlindol hydrochloride with sodium alginate (7.33%, w/w).

**1 - COMPOSITION**

The unitary qualitative and quantitative composition for pirlindol hydrochloride tablets dosed for 100 mg, obtained by the dry granulation process, is shown in Table 3.

TABLE 3 -

| Unitary qualitative and quantitative composition for pirlindol hydrochloride tablets dosed for 100 mg, obtained by the dry granulation process | | |
|---|---|---|
| **Constituents** | **Amount (mg)** | **Function** |
| Pirlindol.HCl | 100.00 | Active substance |
| Sodium alginate | 37.00 | Polymer for prolonging the release |
| Cellactose® (lactose monohydrate + microcrystalline cellulose | 355.40 | Diluent |
| Magnesium stearate | 10.10 | Lubricant |
| Aerosil 200®(anhydrous colloidal silica) | 2.50 | Gliding agent |
| **Mass of 1 Tablet** | **505.0** | **--** |

**2- PREPARATION METHOD**

The pirlindol hydrochloride is mixed with *Cellactose*®. Subsequently sodium alginate is added. Finally the *Aerosil 200*® and the magnesium stearate are added. The obtained mixture is compressed to pre-tablets. The pre-tablets are then divided and calibrated by nets with an appropriate mesh crevice. The obtained granulate is compressed to 14 mm oblong tablets.

### 3 - DISSOLUTION PROFILES

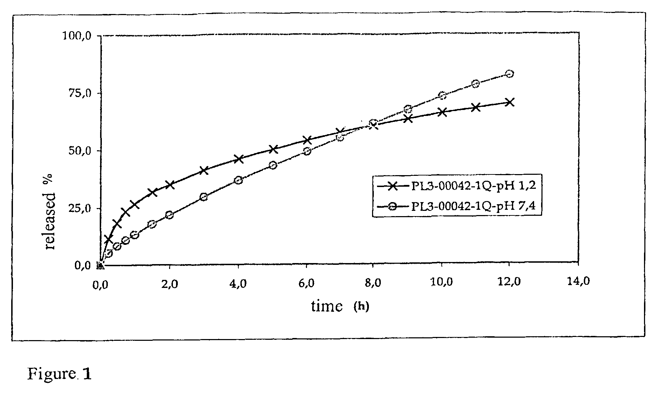

There were carried out dissolution profiles in acid medium (pH 1.2) and in alkaline medium (pH 7.4). The results are shown in Fig. 1.

Surprisingly, it was verified that the sodium alginate itself shows the capability of prolonging the release of pirlindol hydrochloride in mediums with different pH values (Fig. 1), when the used manufacture process corresponds to dry granulation.

Also unexpected was the existence of similarity between the dissolution values of the pirlindol hydrochloride long release tablets in dissolution mediums with extreme pH values (pH 1.2 and pH 7.4).

The similarity factor ($f_2$) was calculated according to equation 1 and the obtained value was 51.4%. The similarity condition is represented by equation 2.

$$\textbf{Eq. 1} \quad f_2 = 50 \cdot \log\left( \frac{1}{\sqrt{1 + meanF}} \cdot 100 \right)$$

$f_2$ - similarity coefficient

$$F - (F_1 - F_2)^2$$

$F_1$ - Dissolved percentage of formulation 1
$F_2$ - Dissolved percentage of formulation 2

$$\text{Eq. 2} \qquad 50 \leq f_2 \leq 100$$

**Example 3:** Preparation of long release tablets, dosed for 100 mg of pirlindol hydrochloride with sodium alginate (15.00%, w/w).

### 1 - COMPOSITION

The unitary qualitative and quantitative composition for pirlindol hydrochloride tablets dosed for 100 mg, obtained by the dry granulation process, is shown in Table 4.

TABLE 4 -

| Unitary qualitative and quantitative composition for pirlindol hydrochloride tablets dosed for 100 mg, obtained by the dry granulation process | | |
|---|---|---|
| **Constituents** | **Amount (mg)** | **Function** |
| Pirlindol.HCl | 100.00 | Active substance |
| Sodium alginate | 75.75 | Polymer for prolonging the release |
| Microcrystalline cellulose | 320.20 | Diluent |
| Magnesium stearate | 6.50 | Lubricant |
| Aerosil 200®(anhydrous colloidal silica) | 2.50 | Gliding agent |
| **Mass of 1 Tablet** | **505.0** | **--** |

### 2- PREPARATION METHOD

The pirlindol hydrochloride is mixed with the microcrystalline cellulose. Subsequently sodium alginate is added. Finally the *Aerosil 200*® and the magnesium stearate are added. The obtained mixture is compressed to pre-tablets. The pre-tablets are then divided and calibrated by nets with an appropriate mesh crevice. The obtained granulate is compressed to 14 mm oblong tablets.

**3 - DISSOLUTION PROFILES**

In order to study the effect of humidity on the long release profiles, the tablets of this formulation were put in ovens at different temperatures and humidity degrees (25°C/60%RH, 40°C/75%RH and 60°C) without any packing material.

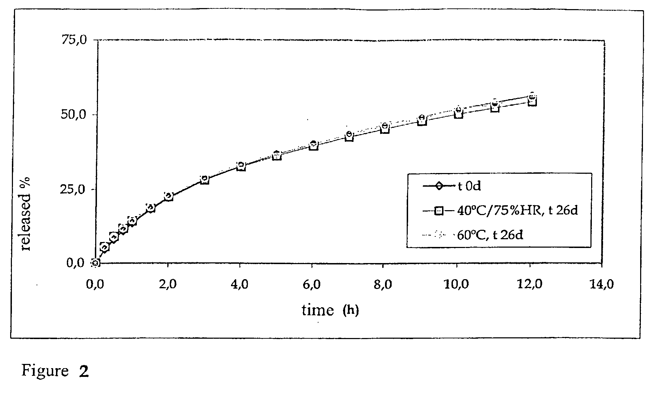

The results after 26 days in the described conditions are shown in Fig. 2.

One of the problems pointed out regarding formulations comprising hydrosoluble alginate derivatives is the instability of the dissolution profiles during the storage period. Surprisingly, for formulations comprising pirlindol hydrochloride and sodium alginate, obtained by the described manufacture process, the profiles are kept stable, even when the formulations are stored at different humidity and temperature conditions.

The $f_2$ value obtained between the profile of tablets in stability at 40°C/75%RH and the release profile at time zero was 92.6% and the $f_2$ value obtained between the profile of tablets in stability at 60°C and the release profile at time zero was 97.2%. The similarity values obtained show a great stability of the dissolution profiles.

**Example 4:** Preparation of long release tablets, dosed for 200 mg of pirlindol hydrochloride with sodium alginate (7.33%, w/w).

**1 - COMPOSITION**

The unitary qualitative and quantitative composition for pirlindol hydrochloride tablets dosed for 200 mg, obtained by the dry granulation process, is shown in Table 5.

TABLE 5 -

| Unitary qualitative and quantitative composition for pirlindol hydrochloride tablets dosed for 200 mg, obtained by the dry granulation process | | |
|---|---|---|
| **Constituents** | **Amount (mg)** | **Function** |
| Pirlindol.HCl | 200.00 | Active substance |
| Sodium alginate | 74.00 | Polymer for prolonging the release |
| Cellactose® (lactose monohydrate + microcrystalline cellulose | 710.80 | Diluent |
| Magnesium stearate | 20.20 | Lubricant |
| Aerosil 200®(anhydrous colloidal silica) | 5.00 | Gliding agent |
| **Mass of 1 Tablet** | **1010.0** | **--** |

**2- PREPARATION METHOD**

The pirlindol hydrochloride is mixed with *Cellactose®.* Subsequently sodium alginate is added. Finally the *Aerosil 200®* and the magnesium stearate are added. The obtained mixture is compressed to pre-tablets. The pre-tablets are then divided and calibrated by nets with an appropriate mesh crevice. The obtained granulate is compressed to 20 mm oblong tablets.

**3 - DISSOLUTION PROFILES**

Dissolution profiles in mediums with different pH values (1.2<pH<7.4) were carried out:

The similarity factors ($f_2$) were calculated for the dissolution profiles at pH 1.2 and pH 7.4, pH 6.8 and pH 7.4 and water and pH 7.4. The results are shown in Table 6.

TABLE 6 -

| Similarity factors | |
|---|---|
| **pH Relation** | **Similarity Factor -$f_2$ (%)** |
| pH 1.2 vs. pH 7.4 | 54.5 |
| pH 6.8 vs. 7.4 | 56.9 |
| Water vs. pH 7.4 | 54.1 |

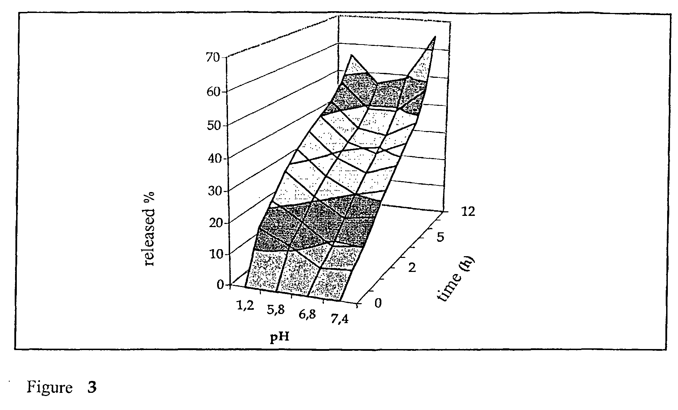

The similarity factors (Table 6) and topographic characterization of the dissolution (Fig. 3) show that the present formulation allows the obtention of similar dissolution profiles in mediums with different pH values.

**Claims**

1. Pharmaceutical compositions of pirlindol hydrochloride and alginic acid derivatives and/or derivatives of an alginic acid salt obtainable by dry mixing and/or dry granulation, which allow prolonging the release of pirlindol hydrochloride during a period of time selected within the range of 8 to 24 hours.

2. Pharmaceutical compositions according to claim 1, **characterized in that** they show stable and similar long dissolution profiles in mediums with a pH value in the range of 1.0≤pH<8.0.

3. Pharmaceutical composition according to claim 1, **characterized in that** the alginic acid derivative is sodium alginate or calcium alginate.

4. Pharmaceutical composition according to claim 1, **characterized in that** it comprises pirlindol hydrochloride, at least one or more alginic acid derivatives and/or derivatives of an alginic acid salt and technologically appropriate excipients to allow the obtention of matricial pharmaceutical forms.

5. Pharmaceutical composition according to claim 1, **characterized in that** the pirlindol hydrochloride is comprised within the range of 2.0% (w/w) to 60% (w/w) of the pharmaceutical composition.

6. Pharmaceutical composition according to claim 1, **characterized in that** the alginic acid derivative is comprised within the range of 1.0% (w/w) to 50% (w/w) of the pharmaceutical composition.

7. Pharmaceutical composition according to claim 1, **characterized in that** the pharmaceutical forms obtained are tablets or grains, for example pellets, for oral administration.

8. Process for the obtention of solid pharmaceutical formulations by dry mixing and/or dry granulation, wherein mixtures of pirlindol hydrochloride with at least one or more alginic acid derivatives and/or derivatives of an alginic acid salt and technologically appropriate excipients are obtained, followed by a pre-compression and utilization of the obtained grains without any additional processing or for the obtention of tablets that yield stable and similar long dissolution profiles during the storage period.

9. Use of pharmaceutical formulations according to claims 1 to 8, for the manufacture of a medicament destined for improving the treatment of psychiatric diseases using only 1 daily take.

10. Use of pharmaceutical formulations according to claims 1 to 8, for the manufacture of a medicament destined for improving the treatment of psychiatric diseases such as depressive syndromes.

**Patentansprüche**

1. Pharmazeutische Zusammensetzungen von Pirlindolhydrochlorid und Alginicsäure-Derivate und/oder Derivate eines Salzes der Alginicsäure erhältlich durch Trockenmischung und/oder Trockengranulierung, welche die Verlängerung der Freisetzung von Pirlindolhydrochlorid während eines Zeitraums im Bereich von 8 bis 24 Stunden erlauben.

2. Pharmazeutische Zusammensetzungen gemäß Anspruch 1, **dadurch gekennzeichnet, daß** sie stabile und ähnliche verlängerte Auflösungsprofile in Mediums mit einem pH-Wert im Bereich von 1.0≤pH<8.0 aufweisen.

3. Pharmazeutische Zusammensetzung gemäß Anspruch 1, **dadurch gekennzeichnet, daß** das Alginicsäure-Derivat Natrium-Alginat oder Calcium-Alginat ist.

4. Pharmazeutische Zusammensetzung gemäß Anspruch 1, **dadurch gekennzeichnet, daß** sie Pirlindolhydrochlorid, mindestens ein oder mehrere Alginicsäure-Derivate und/oder Derivate eines Salzes der Alginicsäure und technologisch geeignete Bestandteile zur Herstellung pharmazeutischer Matrixformen enthält.

**5.** Pharmazeutische Zusammensetzung gemäß Anspruch 1, **dadurch gekennzeichnet, daß** das Pirlindolhydrochlorid in einen Bereich von 2.0 Gew.% bis 60 Gew.% bezogen auf die pharmazeutische Zusammensetzung, enthalten ist.

**6.** Pharmazeutische Zusammensetzung gemäß Anspruch 1, **dadurch gekennzeichnet, daß** das Alginicsäure-Derivat in einen Bereich von 1.0 Gew.% bis 50 Gew.% bezogen auf die pharmazeutische Zusammensetzung, enthalten ist.

**7.** Pharmazeutische Zusammensetzung gemäß Anspruch 1, **dadurch gekennzeichnet, daß** die erhaltenen pharmazeutischen Formen Tabletten oder Granulate, z.B. Pellets, für orale Verabreichung, sind.

**8.** Verfahren zur Herstellung fester pharmazeutischer Formulierungen durch Trockenmischung und/oder Trockengranulierung, wobei Mischungen von Pirlindolhydrochlorid mit mindestens einem oder mehreren Alginiesäure-Derivaten und/oder Derivate eines Salzes der Alginicsäure und technologisch geeignete Bestandteile erhalten werden, und anschließender Vorpressung und Verwendung der erhaltenen Granulate ohne weitere Verarbeitung oder zur Herstellung von Tabletten, die stabile und ähnliche verlängerte Auflösungsprofile während der Lagerung aufweisen.

**9.** Verwendung der pharmazeutischen Formulierungen gemäß Ansprüche 1 bis 8, zur Herstellung eines Arzneimittels zur Verbesserung der Behandlung von psychiatrischen Krankheiten mittels nur einer Tageseinnahme.

**10.** Verwendung der pharmazeutischen Formulierungen gemäß Ansprüche 1 bis 8, zur Herstellung eines Arzneimittels zur Verbesserung der Behandlung von psychiatrischen Krankheiten, wie depressive Syndrome.

**Revendications**

**1.** Compositions pharmaceutiques de chlorhydrate de pirlindol et de dérives d'acide alginique et/ou dérives d'un sel d'acide alginique susceptible d'être obtenu par mélange à sec et/ou granulation à sec, que permettent de prolonger la libération du chlorhydrate de pirlindol durant une période de temps choisie dans l'intervalle entre 8 et 24 heures.

**2.** Compositions pharmaceutiques suivant la Revendication 1, **caractérisées par le fait qu'**elles présentent des profils de dissolution prolongée stables et similaires dans des milieux a une valeur de pH dans l'intervalle $1{,}0 \leq pH < 8{,}0$.

**3.** Composition pharmaceutique suivant a la Revendication 1, **caractérisée par le fait que** le dérive d'acide alginique est l'alginate de sodium où l'alginate de calcium.

**4.** Composition pharmaceutique suivant a la Revendication 1, **caractérisée par le fait qu'**elle comprend le chlorhydrate de pirlindol, au moins un au plusieurs dérives d'acide alginique et/ou dérives d'un sel d'acide alginique et des excipients appropries sur le plan technologique pour permettre l'obtention de formes pharmaceutiques matricielles.

**5.** Composition pharmaceutique suivant a la Revendication 1, **caractérisée par le fait que** le chlorhydrate de pirlindol est compris entre 2,0% (m/m) et 60% (m/m) de la composition pharmaceutique.

**6.** Composition pharmaceutique suivant Revendication 1, **caractérisée par le fait que** le dérive d'acide alginique est compris entre 1,0% (m/m) et 50% (m/m) de la composition pharmaceutique.

**7.** Composition pharmaceutique suivant a la Revendication 1, **caractérisée par le fait que** les formes pharmaceutiques obtenues sont des comprimes ou des granules, tels que les pellets, destines a l'administration par voie orale.

**8.** Procède d'obtention de formulations pharmaceutiques solides par mélange à sec et/ou granulation à sec, où mélanges de chlorhydrate de pirlindol avec au moins un au plusieurs dérives d'acide alginique et/ou des dérives d'un sel d'acide alginique et des excipients appropries sur le plan technologique sont obtenus, et suivis par une precompression et l'utilisation du granule obtenu sans aucun traitement supplémentaire ou pour l'obtention de comprimes qui donnent des profils de dissolution prolongée stables et similaires pendant la période de conservation.

**9.** Utilisation des formulations pharmaceutiques suivant les Revendications 1 a 8, pour la fabrication d'un médicament destine à améliorer le traitement des maladies psychiatriques au moyen d'une seule prise quotidienne.

**10.** Utilisation des formulations pharmaceutiques suivant les Revendications 1 a 8, pour la fabrication d'un médicament destine à améliorer le traitement des maladies psychiatriques, tels que syndromes dépressifs.

Figure. 1

EP 1 300 140 B1

Figure 2

12

Figure 3